# EUROPÄISCHE PATENTANMELDUNG

(11) **EP 0 995 793 A1**
(43) Veröffentlichungstag der Anmeldung: **26.04.2000**
(21) Anmeldenummer: 99106210.0
(22) Anmeldetag: 12.04.1999
(51) Int. Cl.: C12M 1/04, C12M 1/24, C12M 3/06

(54) **Vorrichtung zur Kultivierung und Konzentrierung nicht adhärenter Zellen sowie zur Kokultur zweier unterschiedlicher Zellsorten**

(30) Priorität: 25.09.1998 DE 19844154
(71) Anmelder: GSF-Forschungszentrum für Umwelt und Gesundheit, GmbH, 85764 Oberschleissheim (DE)
(72) Erfinder: Lodri, Antal, 81243 München (DE); Kremer, Jean-Pierre, Dr., 86853 Langgeringen 23 (DE); Reisbach, Gilbert, Dr., 80637 München (DE); Liu, Guoquing, Prof. Dr., Dpmt. of Clinical and, Yi Xue Yun Lu 1, Chongquing 400046 (CN)
(74) Vertreter: Reinhard - Skuhra - Weise & Partner

(57) **Zusammenfassung**

Eine Vorrichtung zur Kultivierung und Konzentrierung nicht adhärenter Zellen sowie zur Kokultur zweier unterschiedlicher Zellsorten besteht zur Vermeidung von Zellaggregationen und - schädigungen und Verringerung des Kontaminationsrisikos aus einem länglichen Hohlgefäß 10 mit einer parallel zu seiner Längsachse L vorgesehenen Liegefläche 17 zur Plazierung des Hohlgefäßes 10 für einen Zellzüchtungsbetrieb, einer senkrecht zu seiner Längsachse L vorgesehenen Standfläche 14 zur Plazierung des Hohlgefäßes 10 für einen Zell-Erntebetrieb oder für eine Kokultur zweier Zellsorten, wenigstens einer verschließbaren Beschickungs- und Entleerungsöffnung 12, die an dem Hohlgefäß 10, an dessen der Standfläche 14 gegenüber liegenden oberen Bereich 13 gebildet ist, und mit einer sich im Hohlgefäß 11 von der Standfläche 14 erstreckenden Trennwand 19, die dessen Inneres teilweise unterteilt und wenigstens im Bereich nahe der Standfläche 14 einen Durchlaß 20 mit einer flüssigkeitsdurchlässigen, jedoch zellundurchlässigen mikroporösen Membran 21 bzw. zellundurchlässigem Sieb aufweist.

## Beschreibung

Die Erfindung betrifft eine Vorrichtung zur Kultivierung und Konzentrierung nicht adhärenter Zellen sowie zur Kokultur zweier unterschiedlicher Zellsorten, die adhärent oder nicht adhärent sind.

Bei der Zellkultivierung werden Zellen in Kulturgefäße mit Nährmedium eingebracht, wobei sie je nach Zelltyp entweder adhärent d.h. an der Oberfläche des Kulturgefäßes, oder aber nicht adhärent, d.h. in Suspension im Nährmedium wachsen. Für die Ernte und die Weiterzucht nicht adhärent wachsender Zellen ist es bisher üblich, die Zellen nach dem Züchtungsvorgang und vor der weiteren Verwendung durch Zentrifugieren zu konzentrieren. Dabei besteht das Problem, daß durch die Zentrifugierung unerwünscht eine Zellaggregation und eine Zellschädigung auftritt. Außerdem entsteht bei der Manipulation ein hohes Kontaminationsrisiko durch Bakterien und Pilze.

Bei der bisher bekannten Technik der Kokultur von zwei Zellsorten werden mit einer flüssigkeits- aber nicht zelldurchlässigen Membran versehene mobile Einsätze verwendet, die in Kulturschalen eingesetzt werden und diese in zwei Zellkompartimente unterteilen. Mit den vorbekannten Einsätzen in Kulturschalen ist jedoch die Kokultivierung von zwei Zellsorten nur im kleinen Maßstab und nur mit ungleichen Bedingungen möglich, da sich eine Zellsorte in der Gewebekulturschale und die andere auf der Membran befindet. Nachteilig ist auch, daß der Austausch des Kulturmediums, das Sammeln des Kulturüberstandes und das wiederholte Einstellen der Zellkonzentration nicht oder nur sehr eingeschränkt möglich ist.

Aus der WO 96/00780 ist eine kompartimentalisierte Zellkulturvorrichtung bekannt, bei der das Zellkompartiment von einer unteren gasdurchlässigen und von einer oberen nährstoffdurchlässigen Membran begrenzt wird, um eine bessere Versorgung der Zellen bei gleichzeitiger Konzentration der hochmolekularen Zellprodukte im Zellkompartiment zu ermöglichen. Diese Zellkulturvorrichtung erlaubt zwar einen Austausch des Zellkulturmediums ohne Zentrifugation, nicht aber die Ernte der hochmolekularen Zellprodukte ohne anschließende Abtrennung der Zellen durch Zentrifugation. Auch eine Kokultur von zwei Zellsorten ist mit dieser Vorrichtung nicht möglich.

Der Erfindung liegt die Aufgabe zugrunde, eine konstruktiv einfach gestaltete Vorrichtung der eingangs genannten Gattung verfügbar zu machen, mit der bei vermindertem Kontaminationsrisiko eine Zellgewinnung ohne Zentrifugation sowie eine einfache Einstellung der Zellkonzentration ermöglicht wird.

Erfindungsgemäß wird diese Aufgabe durch die im Patentanspruch 1 genannten Merkmale gelöst. Bevorzugte Merkmale, die die Erfindung vorteilhaft weiterbilden, sind den nachgeordneten Patentansprüchen zu entnehmen.

Aufgrund der erfindungsgemäßen Gestaltung der Vorrichtung mit fest eingebauter partieller Trennwand wird in günstiger Weise ein partielles Zweikammersystem verfügbar gemacht, bei der vorteilhaft im Erntebetrieb der Zellfreiüberstand durch die Membran auf die andere Kammerseite fließen und dann die gewünschte Menge an Zellüberstand entnommen werden kann, wobei danach dann frisches Medium zuführbar ist. Während sich die Trennwand in Züchtungsbetrieb aufgrund der Plazierung des Hohlgefäßes vorzugsweise über der Zellsuspension befindet, kann somit ohne Zentrifugation nach Senkrechtstellen des Hohlgefäßes nicht nur ein Austausch des Kulturmediums, sondern auch eine Einstellung der Zellkonzentration und ein Sammeln des Kulturüberstandes erfolgen. Dabei werden Zellaggregation und Zellschädigung weitgehend vermieden, und es wird die Einstellung der Zellkonzentration durch einen kontrollierten Medienaustausch erleichtert. Hinzu kommt, daß weniger Manipulation an der Zellkultur erfolgt und damit das Kontaminationsrisiko reduziert wird.

Die erfindungsgemäße Vorrichtung eignet sich konstruktionsbedingt für die Kultur hoher Zellkonzentrationen mit häufigem und einfachem Mediumwechsel und erlaubt in der senkrechten Orientierung die Kokultur von großen Zellzahlen zweier Sorten mit absolut gleichen Kulturbedingungen.

Als besonderer Vorteil der Erfindung sind nicht nur das Schonen der Zellen durch Wegfall der zentrifugationsbedingten Gravitationskräfte und ein schneller und leichter Medienaustausch anzusehen, sondern auch der Umstand, daß ein zellfreier Kulturüberstand einfacher geerntet werden kann als mit dem herkömmlichen Zentrifugieren und anschließendem Filtrieren. Zudem existiert bei Verwendung der erfindungsgemäßen Vorrichtung kein oder ein geringerer Zellverlust als bei der Zentrifugation und anschließendem Absaugen oder Abgießen des Kulturüberstandes.

Günstig ist weiterhin, daß die Manipulationsvorgänge vergleichsweise und einfacher ablaufen als beim Stand der Technik. So dauert beispielsweise der Medienaustausch mit einer herkömmlichen Zentrifugation ca. zwölf Minuten, während bei der erfindungsgemäßen Vorrichtung dieser Zeitaufwand sich auf ca. vier Minuten verringert, insbesondere, weil sich die Manipulation auf einen einfachen Kippvorgang von der Liegefläche auf die Standfläche des Hohlgefäßes beschränkt.

Als vorteilhaft ist weiterhin das Verhindern oder Vermindern der Zellaggregation anzusehen, die im Zellpellet bei der Zentrifugation auftreten kann. Zudem existiert vorteilhaft wegen weniger Manipulation auch ein geringeres Kontaminationsrisiko.

Von Vorteil ist weiterhin, daß durch die Verwendung der Vorrichtung die Kultur einer hohen Zellkonzentration bei häufigem und einfachem Mediumwechsel ermöglicht wird. Zudem besteht die Möglichkeit des permanenten Mediumaustausch, beispielsweise bei der Antikörperherstellung oder bei der Expansion von Zellen im großen Maßstab. Günstig ist weiterhin die Möglichkeit der Kokultur von zwei verschiedenen Zellsorten, beispielsweise zur Untersuchung ihrer humoralen Interaktion.

Hinsichtlich der Ausgestaltung der erfindungsgemäßen Vorrichtung ist es für die Funktion und Herstellung günstig, wenn der Durchlaß mit einer mikroporösen Membran oder einem zellundurchlässigen Sieb bündig mit der Trennwand abschließt oder eine seitlich vorstehende Einfassung aufweist. Dabei sind die Membran- oder Siebgröße, -Form, -Material, -Poren, bzw. Maschengröße beliebig nach Bedarf im Rahmen des gewünschten Zweckes, nämlich an Durchlässigkeit für Medium und Zellüberstand, nicht jedoch für Zellen vorzusehen, veränderbar. Dabei ist alternativ statt des wenigstens einem Durchlasses auch alternativ vorteilhaft, die gesamte Trennwand, ggf. mit Ausnahme eines konstruktionsbedingten Randstegs aus Kunststoff, als mikroporöse Membran oder als zellundurchlässiges Sieb abzubilden wodurch bei senkrechter Position des Hohlgefäßes in günstiger Weise ein Flüssigkeitsaustausch beschleunigt wird.

Die flüssigkeitsdurchlässige, jedoch zellundurchlässige Membran bzw. das flüssigkeitsdurchlässige jedoch zellundurchlässige Sieb bestehen vorteilhafterweise aus einem lichtdurchlässigen Material, müssen jedoch nicht transparent sein, während das Hohlgefäß vorzugsweise aus transparenten Material besteht. Hierdurch ist günstigerweise gewährleistet, daß das Hohlgefäß in einem sogenannten Umkehrmikroskop angeschaut werden kann, also mit einer Beleuchtung von oben und einer Positionierung des Hohlgefäßes über dem Objektiv, wobei die Kultur von dem Boden des Hohlgefäßes her angeschaut werden kann. Das Hohlgefäß, bevorzugt auch die Trennwand besteht aus einem inerten, transparenten Material, insbesondere, jedoch nicht ausschließlich aus Polystyrol.

Die bei stehendem Hohlgefäß gesehene Höhe h der Trennwand ist vorteilhaft so dimensionierbar, daß die Zellsuspension bei senkrechter Position des Hohlgefäßes nicht über die Trennwand hinweg in die Aufnahmekammer für den Zellüberstand fließen kann.

Die Trennwand erstreckt sich dabei im Zellzüchtungsbetrieb oberhalb der Zellsuspension und wird von dieser nicht benetzt, während nach dem Aufstellen des Hohlgefäßes auf seine Standfläche die Trennwand dafür sorgt, daß die gezüchtete Zellkultur in dem Kammerabschnitt bleibt, in der die Zellzüchtung stattgefunden hat, während der zellfreie Überstand durch die Membran bzw. das Sieb auf die andere Kammerseite fließen kann. Durch die Höhe h der Trennwand ist in jedem Fall sichergestellt, daß diese gewünschte Trennung auch erreicht wird.

Gemäß einer bevorzugten Ausgestaltung der Erfindung ist das beiderseits der Trennwand in dem Hohlgefäß verfügbare Volumen annähernd gleich groß. Das Hohlgefäß selbst ist bevorzugt flaschenförmig ausgebildet, und seine Beschickungs- und Entleerungsöffnung ist nach einer vorteilhaften weiteren Ausgestaltung der Erfindung in einem spitzen Winkel zur Längsachse des Hohlgefäßes orientiert, um für eine Pipette einen guten Zugang in jede Kammer zu ermöglichen. Weiterhin kann alternativ statt einer Beschickungs- und Entleerungsöffnung auch vorgesehen sein, daß zwei derartige Öffnungen mit funktionaler Zuordnung zu jeder Kammer vorhanden sind.

Die Öffnung bzw. Öffnungen sind nach einer weiteren Ausgestaltung der Erfindung lose mit einer festen Kappe oder mit einer Filterkappe verschließbar, um zu gewährleisten, daß kein gasdichter Verschluß vorliegt und ein Zirkulieren von Gas stattfinden kann.

Nachfolgend wird ein Ausführungsbeispiel der Erfindung unter Bezugnahme auf die beigefügten Zeichnungen näher erläutert. Es zeigen:
- Figur 1: eine schematisierte Vorderansicht auf ein flaschenförmiges Hohlgefäß gemäß der Erfindung;
- Figur 2: das Hohlgefäß von Figur 1 in schematisierter Seitenansicht;
- Figur 3: eine schematisierte Draufsicht auf das Hohlgefäß von Figur 1;
- Figur 4: eine schematische Darstellung des Hohlgefäßes von Figur 1 in liegender Plazierung für einen Zell-Züchtungsbetrieb; und
- Figur 5: eine schematisierte Darstellung des Hohlgefäßes von Figur 1 im stehendem Zustand für einen Zell-Erntebetrieb.

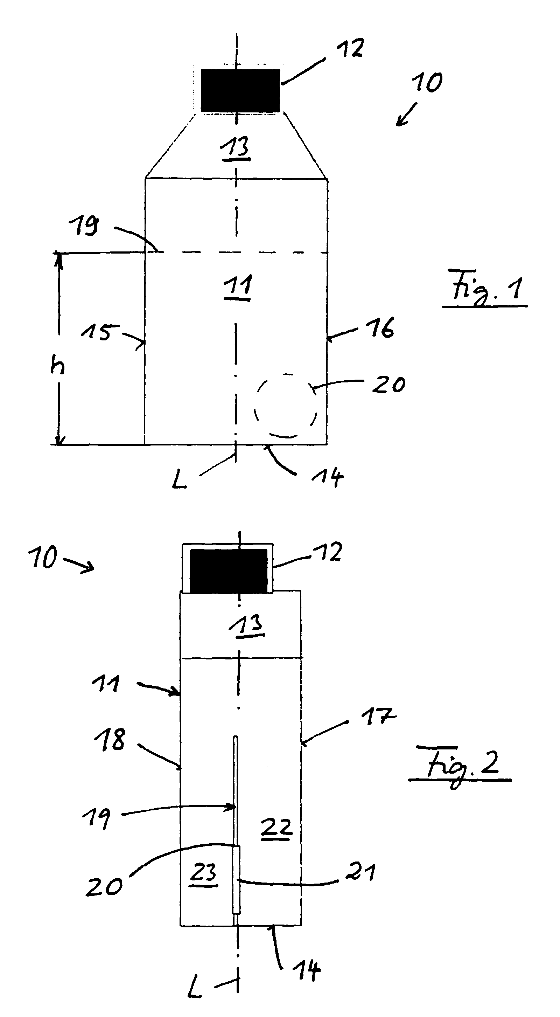

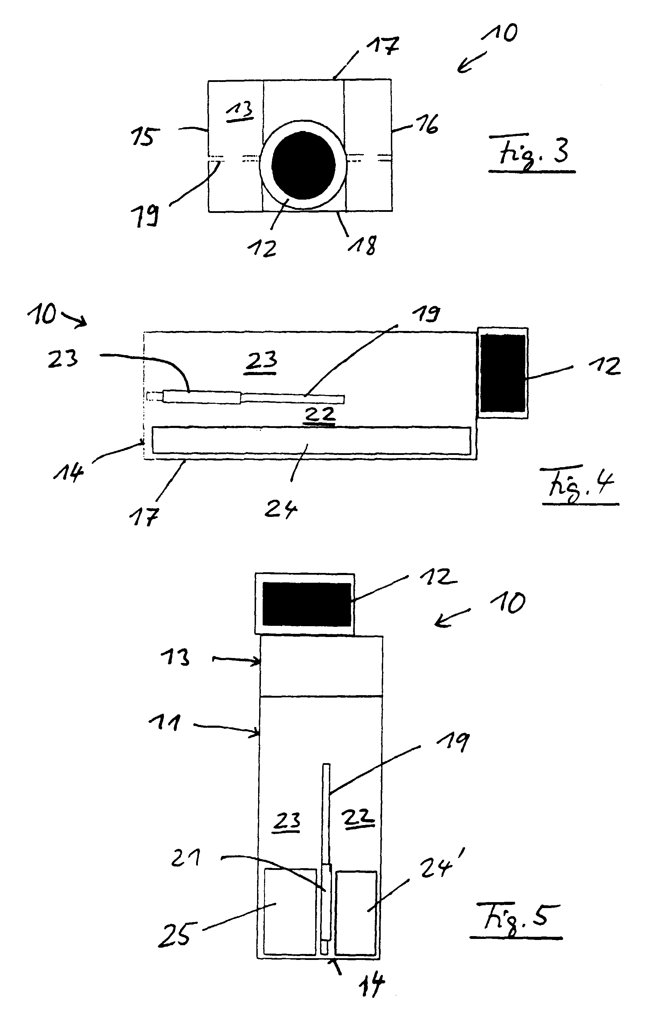

In Figur 1 ist schematisiert eine Ausgestaltung der Vorrichtung als flaschenförmiges Hohlgefäß 10 gezeigt. Das Hohlgefäß 10 weist einen quaderförmigen Hauptkörper 11 mit einem sich daran anschließenden abgewinkelt zu einer Beschickungs- und Entleerungsöffnung 12 verjüngten Abschnitt 13 auf und besitzt eine unterseitige Standfläche 14 und kurz gegenüberliegende Seitenflächen 15 und 16 sowie, wie in Figur 2 erkennbar, größere
parallele Seitenflächen 17 und 18.

Gestrichelt dargestellt ist in Figur 1 eine Trennwand 19, die sich von der Bodenfläche 14 in Richtung auf die Beschickungs- und Entleerungsöffnung 12 parallel zur Längsachse des Hohlgefäßes erstreckt. In der Trennwand 19 ist nahe der Bodenfläche 14 ein kreisförmiger Durchlaß 20 mit einem flüssigkeitsdurchlässigen, jedoch zellundurchlässigen Membranfilter 21 vorgesehen.

Die Trennwand 19 ragt bei dem dargestellten Ausführungsbeispiel über die Hälfte des Hohlgefäßes von der Bodenwand 14 bis zu einer Höhe h nach oben und sorgt für die Schaffung eines partiellen Zweikammersystems, das aus einer Aufnahmekammer 22 für eine Zellsuspension und eine Aufnahmekammer 23 für Zellüberstand besteht. Die Beschickungs- und Entleerungsöffnung 12 ist, wie auch im Zusammenhang mit Figur 3 entnehmbar, außermittig einer Hohlgefäß-Längsachse L angeordnet.

Figur 4 zeigt die Vorrichtung 10 auf ihrer Seitenfläche 17 liegend in einem Zell-Züchtungsbetrieb, bei der die Kammer 22 unterhalb der Kammer 23 liegt und sich die Trennwand 19 parallel zur Grundfläche 17 erstreckt. Die Kammer 22 ist teilweise mit einer Zellsuspension 24 gefüllt, die aus einem Nährmedium mit Zellen besteht.

In der in Figur 5 gezeigten Zell-Erntestellung befindet sich das Hohlgefäß 10 auf seiner Standfläche 14. In dieser Stellung befindet sich in der Kammer 22 ein durch Zellwachstum entstandenes Zellkonzentrat 24', während ein Kulturüberstand 25 durch die Membran 21 in die Kammer 23 zur Abtrennung geströmt ist. Die Kammer 22 und 23 können nun mit einer Pipette entleert werden.

In der in Figur 5 gezeigten Erntestellung ist die Vorrichtung 10 auch zur Kokultur von zwei Zellsorten geeignet.

## Patentansprüche

1. Vorrichtung zur Kultivierung und Konzentrierung nicht adhärenter Zellen sowie zur Kokultur zweier unterschiedlicher Zellsorten, bestehend aus einem länglichen Hohlgefäß (10) mit
einer parallel zu seiner Längsachse (L) vorgesehene Liegefläche (17) zur Plazierung des Hohlgefäßes (10) für einen Zell-Züchtungsbetrieb,
einer senkrecht zu seiner Längsachse (L) vorgesehenen Standfläche (14) zur Plazierung des Hohlgefäßes (10) für einen Zell-Erntebetrieb oder für eine Kokultur zweier Zellsorten,
wenigstens einer verschließbaren Beschickungs- und Entleerungsöffnung (12), die an dem Hohlgefäß (10) an dessen der Standfläche (14) gegenüber liegenden oberen Bereich (13) gebildet ist, und mit
einer sich im Hohlgefäß (10) von der Standfläche (14) erstreckenden Trennwand (19), die dessen Inneres teilweise unterteilt (22, 23), wobei die Trennwand wenigstens teilweise im Bereich nahe der Standfläche (14) mit einer flüssigkeitsdurchlässigen, jedoch zellundurchlässigen Membran (21) bzw. einem zellundurchlässigen Sieb gebildet ist.

2. Vorrichtung nach Anspruch 1,
**dadurch gekennzeichnet,**
daß die Membran (21) bzw. das zellundurchlässige Sieb bündig mit der Trennwand (19) abschließt.

3. Vorrichtung nach Anspruch 1,
**dadurch gekennzeichnet,**
daß der Abschnitt der Trennwand als Durchlaß (20) mit einer seitlich vorstehende Einfassung ausgebildet ist.

4. Vorrichtung nach einem der vorangehenden Ansprüche,
**dadurch gekennzeichnet,**
daß der Durchlaß (20) kreisförmig ausgebildet ist.

5. Vorrichtung nach einem der vorangehenden Ansprüche,
**dadurch gekennzeichnet,**
daß die Membran (21) bzw. das zellundurchlässige Sieb aus PE oder Polycarbonat besteht.

6. Vorrichtung nach einem der vorangehenden Ansprüche,
**dadurch gekennzeichnet,**
daß das Hohlgefäß (10) aus einem transparenten Material besteht.

7. Vorrichtung nach einem der vorangehenden Ansprüche,
**dadurch gekennzeichnet,**
daß die Trennwand (19) aus einem lichtdurchlässigen, vorzugsweise transparenten Material besteht.

8. Vorrichtung nach einem der vorangehenden Ansprüche,
**dadurch gekennzeichnet,**
daß bei stehendem Hohlgefäß (10) die Höhe (h) der Trennwand (19) derart bemessen ist, daß die Zellsuspension in dieser Position an einem Überfließen gehindert ist.

9. Vorrichtung nach einem der vorangehenden Ansprüche,
**dadurch gekenzeichnet,**
daß die Beschickungs- und Entleerungsöffnung (12) lose mit einer festen Kappe oder mit einer gasdurchlässigen Filterkappe verschließbar ist.

10. Vorrichtung nach einem der vorangehenden Ansprüche,
**dadurch gekennzeichnet,**
daß die Beschickungs- und Entleerungsöffnung (12) in einem spitzen Winkel zur Längsachse (1) des Hohlgefäßes (10) orientiert ist.

11. Vorrichtung nach einem der vorangehenden Ansprüche,
**dadurch gekennzeichnet,**
daß die Beschickungs- und Entleerungsöffnung (12) außermittig zur Längsachse (L) des Hohlgefäßes (10) angeordnet ist.

12. Vorrichtung nach einem der vorangehenden Ansprüche,
**dadurch gekennzeichnet,**
daß das beiderseits der Trennwand (19) in dem Hohlgefäß (10) verfügbare Volumen der Kammern (22, 23) annähernd gleich groß ist.

13. Vorrichtung nach einem der vorangehenden Ansprüche,
**dadurch gekennzeichnet,**
daß das Hohlgefäß (10) flaschenförmig ausgebildet ist.
